# EUROPÄISCHE PATENTSCHRIFT

(11) **EP 0 857 065 B1**
(45) Veröffentlichungstag und Bekanntmachung des Hinweises auf die Patenterteilung: **07.04.1999**
(21) Anmeldenummer: 96945354.7
(22) Anmeldetag: 15.10.1996
(51) Int. Cl.: A61K 31/27

(54) **VERWENDUNG VON 2-AMINO-4-(4-FLUORBENZYLAMINO)-1-ETHOXYCARBONYLAMINOBENZEN ZUR PROPHYLAXE UND BEHANDLUNG DER FOLGEN DER AKUTEN UND CHRONISCHEN ZEREBRALEN MINDERDURCHBLUTUNG SOWIE NEURODEGENERATIVER ERKRANKUNGEN**
USE OF 2-AMINO-4-(4-FLUOROBENZYLAMINO)-1-ETHOXY-CARBONYLAMINOBENZENE FOR PROPHYLAXIS AND TREATMENT OF SEQUELAE OF ACUTE AND CHRONIC REDUCED CIRCULATION IN THE BRAIN AND NEURODEGENERATIVE DISORDERS
UTILISATION DE 2-AMINO-4-(4-FLUOROBENZYLAMINO)-1-ETHOXY-CARBONYLAMINOBENZENE POUR LA PROPHYLAXIE ET LE TRAITEMENT DES SEQUELLES D'UNE INSUFFISANCE DE VASCULARISATION CEREBRALE AIGÜE ET CHRONIQUE ET DE MALADIES NEURODEGENERATIVES

(30) Priorität: 26.10.1995 DE 19539861
(43) Veröffentlichungstag der Anmeldung: 12.08.1998
(73) Patentinhaber: ASTA Medica Aktiengesellschaft, D-01277 Dresden (DE)
(72) Erfinder: ROSTOCK, Angelika, D-01129 Dresden (DE); TOBER, Christine, D-01689 Weinböhla (DE); RUNDFELDT, Chris, D-01640 Coswig (DE); BARTSCH, Reni, D-01257 Dresden (DE)
(86) Internationale Anmeldenummer: DE9601951
(87) Internationale Veröffentlichungsnummer: WO9715300

(56) Entgegenhaltungen:
- EP-A- 0 657 427
- WO-A-95/05175
- DE-A- 4 200 259
- EXPERT OPINI. INVEST. DRUGS, Bd. 3, Nr. 12, 1994, Seiten 1331-1332, XP000653354 H. HERDON: "17th Annual Meeting of the European neuroscience association"
- DATABASE WPI Section Ch, Week 9204 Derwent Publications Ltd., London, GB; Class B02, AN 92-030619 XP002029612 & JP 03 279 327 A (TSUMURA & CO) , 10.Dezember 1991
- DATABASE WPI ZUSAMMENFASSUNG NR. 92-350553 XP002029611

## Beschreibung

Die Erfindung betrifft die Verwendung von 2-Amino-4-(4-fluorbenzylamino)-1-ethoxycarbonylaminobenzen der Formel I 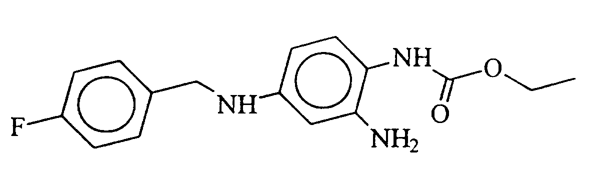 oder deren pharmazeutisch verwendbaren Salze zur Herstellung von Arzneimitteln zur Prophylaxe und Behandlung der Folgen der akuten und chronisch zerebralen Minderdurchblutung und neurodegenerativer Erkrankungen.

Die Verbindung I befindet sich in der Entwicklung als Antikonvulsivum. Es weist ein breites Wirkungssprektrum gegen verschiedene experimentell erzeugte Konvulsionen und in genetischen Tiermodellen auf. Die Wirksamkeit im Tier ist höher als die von vielen eingeführten Antikonvulsiva. Weiterhin sind muskelrelaxierende, fiebersenkende und analgetische Wirkungen beschrieben worden (DE 42 00 259).

Ein Problem vieler eingeführter Antikonvulsiva, vor allem der GABA-verstärkenden Substanzen wie Phenobarbital, Diazepam und Clonazepam aber auch Phenytoin, einem Blocker des Natriumkanals, ist die negative Beeinflussung mentaler Leistungen. Durch die Verstärkung der Hemmung im Gehirn kommt es neben der antikonvulsiven Wirkung auch zu einer zentralen Sedation, die das Aufnahmevermögen der Patienten reduzieren.

Diese Antikonvulsiva sind zudem weder im Tierexperiment noch beim Patienten neuroprotektiv wirksam. Die Folgen einer zerebralen Minderdurchblutung, wie sie beispielsweise beim Schlaganfall auftritt, werden nicht abgeschwächt.

Im epileptischen Anfall kommt es auch zu einer Unterversorgung der betroffenen Hirngebiete, die jedoch nicht auf eine Minderdurchblutung, sondern auf die starke Zellaktivierung zurückzuführen ist, wodurch die Reserven belastet werden und die Zufuhr nicht mehr ausreicht.

Deshalb ist ein Antikonvulsivum, das im belasteten Gehirn eine neuroprotektive Wirkung entfaltet, wünschenswert.

Eine neuroprotektive Wirkung ist auch für die Therapie anderer neurodegenerative Erkrankungen notwendig. Zu diesen sind beispielsweise der Morbus Alzheimer, die Huntington's Chorea, die Multiple Sklerose, die AIDS-induzierte Encephalopathie und andere infektionsbedingte Encephalopathien die durch Röteln-Viren, Herpes-Viren, Borrelien und durch unbekannte Erreger verursacht werden, die Creutzfeld-Jakob-Erkrankung, die Amyotrophe Lateralsklerose (ALS), der Morbus Parkinson, Trauma-induzierte Neurodegenerationen und neuronale Übererregungszustände wie im Medikamentenentzug oder durch Intoxikationen sowie neurodegenerative Erkrankungen des peripheren Nervensystems wie Polyneuropathien und Polyneuritiden zu zählen.

Zur Behandlung der zerebralen Minderdurchblutung und des Schlaganfalls werden zur Zeit mehrere Strategien verfolgt. Prophylaktisch können Medikamente verwendet werden, die die Thrombusbildung hemmen und die Fließeigenschaften des Blutes steigern wie Acetylsalicylsäure. Eine solche Behandlung weist jedoch nur eine rein prophylaktische Wirkung auf, eine Therapie ist damit nicht möglich.

Wenn eine chronische zerebrale Minderdurchblutung vorliegt, kommen Medikamente zum Einsatz, die gefäßaufweitend wirksam sind wie Calciumantagonisten.

Zur Therapie des Schlaganfalls als akute Minderdurchblutung können auch Präparate eingesetzt werden, die thrombolytisch wirksam sind, um einen eventuellen Gefäßverschluß zu beseitigen. Diese können jedoch nur eingesetzt werden, wenn in eingehenden Untersuchungen eindeutig geklärt ist, daß dem Schlaganfall keine zerebrale Blutung zugrunde liegt. In der klinischen Erprobung zur Therapie des Schlaganfalls befinden sich Präparate mit NMDA-antagonistischer Wirkung, die die Überaktivierung der minderversorgten Zellen direkt hemmen. Diese Substanzen weisen jedoch ein hohes Nebenwirkungspotential auf. Sie können daher nach heutiger Sicht nur unter intensivmedizinischer Betreuung nach eindeutiger Diagnosestellung eingesetzt werden. Zudem weisen NMDA-Antagonisten durch die Hemmung der Plastizität des Gehirns eine negative Wirkung auf die Lernleistung auf. Ein prophylaktischer Einsatz dieser Präparate erscheint daher, trotz der guten prophylaktischen Wirkung im Tierexperiment, aus heutiger Sicht ausgeschlossen.

Aufgabe der vorliegenden Erfindung ist es, ein Arzneimittel mit guten neuroprotektiven Eigenschaften und einem geringen Nebenwirkungspotential zur Prophylaxe und Behandlung des Schlaganfalls, der zerebralen Minderdurchblutung und anderen Nervenzell-belastenden Zuständen bereitzustellen.

Überraschenderweise wurde nun gefunden, daß die Verbindung I bedeutende neuroprotektive Wirkungen im Tierexperiment aufweist.

Damit eröffnen sich völlig neue Möglichkeiten für die Prophylaxe und Behandlung der Folgen der akuten und chronischen zerebralen Minderdurchblutung, insbesondere des Schlaganfalls, sowie für neurodegenerativer Erkrankungen.

### Pharmakologische Untersuchungen:

Ziel der Untersuchung mit der Verbindung I in Modellen zur Lernleistung und Neuroprotektion war, die mögliche Beeinflussung dieser Parameter abzuschätzen, da die Verbindung I unter anderem eine GABA-erge Wirkung entfaltet. Da der Epilepsiepatient durch die wiederholten Anfälle oft bereits unter einem Lernleistungsdefizit leidet, wurden diese Versuche an Tieren durchgeführt, denen ein amnestischer Faktor gesetzt worden war, die also in ihrer Lernleistung gemindert waren. Dazu wurden die Tiere entweder wiederholt mit Elektroschocks behandelt oder einem Alkoholentzug ausgesetzt; zur Abschätzung der direkten neuroprotektiven Wirkung wurde bei den Tieren eine chronischen Minderdurchblutung des Gehirns durch Abbinden von zuführenden Blutgefäßen hervorgerufen. Alle diese Schädigungen führen zu einer Reduktion der Lernleistung, was als Indikator einer Nervenzellschädigung zu werten ist. In diesen Modellen weisen GABA-verstärkende antiepileptisch wirksame Medikamente wie Diazepam und Natriumkanalblocker wie Phenytoin keine positiven Effekte auf, in höheren Dosierungen können sogar negative Effekte auf die Lernleistung auftreten.

### Untersuchungsmodelle:

### Lernleistungsschädigung durch Verringerung der Durchblutung des Gehirns

In diesem Modell werden Ratten unter Narkose eine der Halsschlagadern (Carotiden) abgebunden.
Die Tiere erwachen aus der Narkose und weisen danach eine verringerte Lernleistung auf. Diese wurde mittels des Stabsprungtests festgestellt. Bei diesen Versuchen müssen die Tiere lernen, einem leichten elektrischen Fußschock, der ihnen durch ein akustisches Signal vorher angekündigt wird, durch einen Sprung an einen über dem Fußboden hängenden senkrechten Stab auszuweichen.
Die Lernleistung der Tiere wird in Anzahl der bedingten Reaktionen (Sprung an den Stab während der Phase des akustischen Signals) in Prozent gemessen.

Unbehandelte und zum Schein operierte Tiere (in Narkose gelegt und Gefäße freigelegt, aber keine Ligatur vorgenommen) lernen die Verbindung vom akustischen Signal und dem darauf folgenden unangenehmen Fußschock sehr schnell. Nach 4 Versuchstagen mit täglich 10 Expositionen reagieren die Tiere fast bei jedem Tonsignal mit einem Sprung an den senkrechten Stab.
Durch die Ligatur der linken Carotis wird diese Lernleistung etwa auf die Hälfte reduziert.
Mit 2 mg/kg i.p. der Verbindung I eine Stunde vor jeder Versuchsphase vorbehandelte Tiere lernten trotz bestehender Schädigung durch die Ligatur unerwarteter Weise genau so gut, tendenziell sogar besser als nicht operierte Tiere. Wurden die Tiere jedoch mit Diazepam(0,3 mg/kg i.p., 1 Stunde vor jeder Trainingsphase) vorbehandelt, dann blieb die Lernleistung ähnlich schlecht wie bei den unbehandelten geschädigten Tieren.

Ähnliches gilt für eine Behandlung mit dem Antikonvulsivum Phenytoin (3 und 10 mg/kg), die Lernleistung konnte nicht verbessert werden.

Eine Verbesserung der Lernleistung trotz der bestehenden Minderdurchblutung ist als Indikator einer zellschützenden Wirkung anzusehen, da nur voll funktionstüchtige Nervenzellen lernfähig sind.

Es ist daher zu erwarten, daß die Verbindung I eine zellschützende Wirkung beispielsweise im Randbereich eines Infarktes, wo auch eine Minderdurchblutung vorliegt oder auf belastete Zellen, die einem relativem Energiemangel unterliegen, ausübt. Dadurch sollte das Infarktvolumen und damit die Schäden geringer bleiben und stark belasteten Zellen das Überleben ermöglicht werden.

**Tabelle 1**

| Anzahl der bedingten Reaktionen in % im Stabsprung nach Schädigung durch Ligatur der linken Carotis. | | | | |
|---|---|---|---|---|
| Carotis ligatur links | 1. Tag | 2. Tag | 3. Tag | 4. Tag |
| Verbindung I 2 mg/kg | 20 ± 2,6 | 59 ± 7,7 * | 64 ± 9,1 * | 70 ± 6,5 ** |
| Kontrolle Ligatur | 16 ± 2,2 | 30 ± 3,7 ⁺⁺ | 36 ± 3,1 ⁺⁺ | 39 ± 3,8 ⁺⁺ |
| Kontrolle Scheinligatur | 18 ± 2,0 | 55 ± 6,9 | 59 ± 5,9 | 62 ± 5,9 |
| Diazepam 0,3 mg/kg | 9 ± 1,8 | 37 ± 4,2 * | 38 ± 5,1 | 44 ± 5,0 |
| Kontrolle Ligatur | 13 ± 3,0 ⁺ | 30 ± 2,1 ⁺⁺ | 37 ± 2,6 ⁺⁺ | 38 ± 3,6 ⁺⁺ |
| Kontrolle Scheinligatur | 21 ± 2,3 | 52 ± 5,1 | 64 ± 4,8 | 71 ± 3,8 |
| Phenytoin 10 mg/kg | 13 ± 2,1 | 38 ± 4,2 | 45 ± 5,6 | 48 ± 4,4 * |
| Phenytoin 3 mg/kg | 14 ± 1,6 | 38 ± 3,6 | 39 ± 4,1 | 42 ± 4,7 |
| Kontrolle Ligatur | 13 ± 3,0 ⁺ | 30 ± 2,1 ⁺⁺ | 37 ± 2,6 ⁺⁺ | 38 ± 3,6 ⁺⁺ |
| Kontrolle Scheinligatur | 21 ± 2,3 | 52 ± 5,1 | 64 ± 4,8 | 71 ± 3,8 |
| Signifikante Unterschiede zwischen der scheinoperierten Kontrollgruppe und der Kontrollgruppe mit Ligatur (t-Test) sind markiert mit | | | | |

| | | | | |
|---|---|---|---|---|
| ⁺ p<0,05 und | | | | |
| ++ p<0,01. Signifikante Unterschiede zwischen der Kontrollgruppe mit Ligatur und der behandelten Gruppe sind gekennzeichnet mit | | | | |
| * p<0,05 und | | | | |
| ** p<0,01. | | | | |

Die Verbindung I wies nicht nur in diesem Modell eine ausgezeichnete Wirkung auf, auch die durch wiederholte Applikation von Elektroschocks hervorgerufene Lernleistungsminderung konnte durch eine Vorbehandlung mit 2 mg/kg der Verbindung I eine Stunde vor dem Versuch reduziert werden.

Während am 4. Versuchstag geschädigte Versuchstiere nur 32 ± 2,9 % bedingte Reaktionen zeigten, konnten die behandelten Tiere 45 ± 4,5 % bedingte Reaktionen richtig ausführen.
Diese Wirkung war auch nach einer Vorbehandlungszeit von 2 Stunden nachweisbar. Die Anzahl der bedingten Reaktionen stieg hier von 35 ± 3,7 % in der Kontrollgruppe auf 52 ± 3,9 % in der behandelten Gruppe.

Auch die Lernleistungsminderung durch Alkoholentzug konnte positiv beeinflußt werden.

Die Verbindung I kann damit als hoch spezifischer Wirkstoff zur Behandlung der Folgen der akuten und chronischen zerebralen Minderdurchblutung, insbesondere des Schlaganfalls, sowie bei allen Zuständen während und nach Belastung von Nervenzellen eingesetzt werden.

Aufgrund der geringen Nebenwirkungen der Substanz im Tierexperiment kann die Verbindung I auch zur Prophylaxe der oben genannten Erkrankungen und Zustände eingesetzt werden.

Die Verbindung I ist strukturell verwandt mit Flupirtin, einem klinisch eingeführten zentralen Analgetikum. Während bei Flupirtin eine NMDA-antagonistische Wirkung gefunden wurde (WO 95/05175), konnte eine solche für die Verbindung I in in vitro- Experimenten ausgeschlossen werden.

Es wurde weder eine Affinität zu den verschiedenen Bindungsstellen des NMDA-Rezeptors noch eine direkte Beeinflussung des durch NMDA ausgelösten Stroms gefunden.

In vertiefenden Untersuchungen zur zentralen analgetischen Wirkung der Verbindung I im Hot Plate Test konnte im Gegensatz zu Flupirtin eine zentral analgetische Wirkung, wie sie im Hot Plate-Test an der Maus für Flupirtin mit einer mittleren wirksamen Dosis von 30 mg/kg nachgewiesen wurde, ausgeschlossen werden.

NMDA-Antagonisten können schwere psychotische Störungen, wie Ataxie mit stereotypen Erscheinungen hervorrufen.

Die Verbindung I und Verfahren zu ihrer Herstellung sind bekannt (DE 42 00 259).

Die Verbindung kann in bekannter Weise in die üblichen Formulierungen wie Tabletten, Kapseln, Dragees, Pillen, Granulate, Sirupe, Emulsionen, Suspensionen und Lösungen, unter Verwendung inerter, nicht toxischer, pharmazeutisch geeigneter Träger- und/oder Hilfsstoffe überführt werden.

Hierbei sollte die Tagesdosis der Verbindung I bei oraler oder parenteraler Gabe 50-500 mg betragen. Erforderlichenfalls kann von den genannten Mengen abgewichen werden und zwar in Abhängigkeit vom Körpergewicht und der speziellen Art des Applikationsweges.

## Patentansprüche

1. Verwendung der Verbindung I 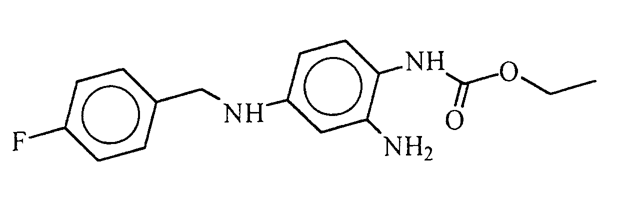 oder deren pharmazeutisch verwendbaren Salze zur Herstellung eines Arzneimittels zur Propylaxe und Behandlung der Folgen der chronischen zerebralen Minderdurchblutung, insbesondere des Schlaganfalls.

2. Verwendung der Verbindungen der Formel I zur Herstellung von Arzneimitteln zur Behandlung neurodegenerativer Erkrankungen.

3. Verwendung der Verbindungen der Formel I gemäß Anspruch 2 zur Herstellung von Arzneimitteln zur Behandlung von Morbus Alzheimer.

4. Verwendung der Verbindungen der Formel I gemäß Anspruch 2 zur Herstellung von Arzneimitteln zur Behandlung von Huntington's Chorea.

5. Verwendung der Verbindungen der Formel I gemäß Anspruch 2 zur Herstellung von Arzneimitteln zur Behandlung von Multipler Sklerose.

6. Verwendung der Verbindungen der Formel I gemaß Anspruch 2 zur Herstellung von Arzneimitteln zur Behandlung von Amyotrophischer Lateralsklerose.

7. Verwendung der Verbindungen der Formel I gemäß Anspruch 2 zur Herstellung von Arzneimitteln zur Behandlung von AIDS-induzierter Encephalopathie und anderen infektionsbedingten Encephalopathien, die durch Röteln-Viren, Herpes-Viren, Borrelien und durch unbekannte Erreger verursacht werden.

8. Verwendung der Verbindungen der Formel I gemaß Anspruch 2 zur Herstellung von Arzneimitteln zur Behandlung der Creutzfeld-Jakob-Erkrankung.

9. Verwendung der Verbindungen der Formel I gemäß Anspruch 2 zur Herstellung von Arzneimitteln zur Behandlung von Morbus Parkinson.

10. Verwendung der Verbindungen der Formel I gemäß Anspruch 2 zur Herstellung von Arzneimitteln zur Behandlung von Trauma-induzierten Neurodegenerationen und neuronalen Übererregungszustände wie im Medikamentenentzug oder durch Intoxikationen.

11. Verwendung der Verbindungen der Formel I gemäß Anspruch 2 zur Herstellung von Arzneimitteln zur Behandlung von neurodegenerativen Erkrankungen des peripheren Nervensystems wie Polyneuropathien und Polyneuritiden.

## Claims

1. Use of the compound I 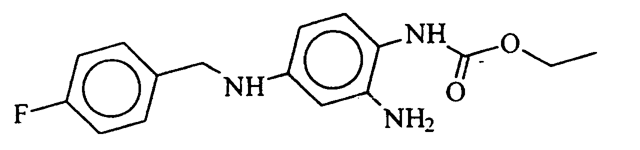 or its pharmaceutically utilizable salts for the production of a medicament for the prophylaxis and treatment of the sequelae of chronic reduced cerebral blood supply, in particular of stroke.

2. Use of the compounds of the formula I for the production of medicaments for the treatment of neurodegenerative disorders.

3. Use of the compounds of the formula I according to Claim 2 for the production of medicaments for the treatment of Alzheimer's disease.

4. Use of the compounds of the formula I according to Claim 2 for the production of medicaments for the treatment of Huntington's chorea.

5. Use of the compounds of the formula I according to Claim 2 for the production of medicaments for the treatment of multiple sclerosis.

6. Use of the compounds of the formula I according to Claim 2 for the production of medicaments for the treatment of amyotrophic lateral sclerosis.

7. Use of the compounds of the formula I according to Claim 2 for the production of medicaments for the treatment of AIDS-induced encephalopathy and other infection-related encephalopathies which are caused by rubella viruses, herpes viruses, borrelia and by unknown pathogens.

8. Use of the compounds of the formula I according to Claim 2 for the production of medicaments for the treatment of Creutzfeld-Jakob disease.

9. Use of the compounds of the formula I according to Claim 2 for the production of medicaments for the treatment of Parkinson's disease.

10. Use of the compounds of the formula I according to Claim 2 for the production of medicaments for the treatment of trauma-induced neurodegenerations and neuronal hyperexcitation states such as in medicament withdrawal or [lacuna] by intoxication.

11. Use of the compounds of the formula I according to Claim 2 for the production of medicaments for the treatment of neurodegenerative disorders of the peripheral nervous system such as polyneuropathies and polyneuritides.

## Revendications

1. Utilisation du composé I, 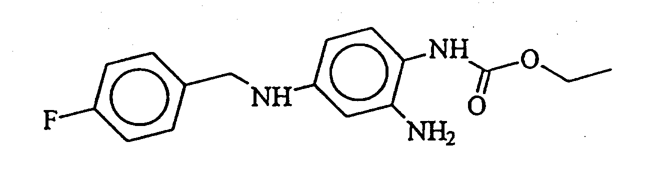 ou de ses sels pharmaceutiquement utilisables pour la fabrication d'un médicament pour la prophylaxie et le traitement des séquelles de la diminution de l'irrigation sanguine cérébrale chronique en particulier de l'attaque.

2. Utilisation des composés de formule I pour la fabrication de médicaments pour le traitement des maladies neurodégénératives.

3. Utilisation des composés de formule I selon la revendication 2 pour la fabrication des médicaments pour le traitement de la maladie d'Alzheimer.

4. Utilisation des composés de formule I selon la revendication 2, pour la fabrication de médicaments pour le traitement de la Chorée d'Huntington.

5. Utilisation des composés de formule I selon la revendication 2, pour la fabrication de médicaments pour le traitement de la sclérose en plaques.

6. Utilisation des composés de formule I selon la revendication 2, pour la fabrication de médicaments pour le traitement de la Sclérose latérale amyotrophique.

7. Utilisation des composés de formule I selon la revendication 2, pour la fabrication de médicaments pour le traitement des encéphalopathies induites par le SIDA et d'autres encéphalopathies conditionnées par une infection qui sont causées par les virus de la rubéole, les virus de l'herpès, les Borrelioses, et par des agents pathogènes inconnus.

8. Utilisation des composés de formule I selon la revendication 2, pour la fabrication de médicaments pour le traitement de la maladie de Creutzfeld-Jakob.

9. Utilisation des composés de formule I selon la revendication 2, pour la fabrication de médicaments pour le traitement de la maladie de Parkinson.

10. Utilisation des composés de formule I selon la revendication 2, pour la fabrication de médicaments pour le traitement de dégénérescences nerveuses induites par un trauma et des états de surexcitation neuronales comme dans la privation de médicaments ou par intoxications.

11. Utilisation des composés de formule I selon la revendication 2, pour la fabrication de médicaments pour le traitement des maladies neurodégénératives du système nerveux périphérique comme les polyneuropathies et les polynévrites.
